(19)

**Europäisches Patentamt**
**European Patent Office**
**Office européen des brevets**

(11) **EP 3 511 716 A1**

(12) **EUROPEAN PATENT APPLICATION**

(43) Date of publication:
**17.07.2019 Bulletin 2019/29**

(51) Int Cl.:
***G01N 33/68*** (2006.01)

(21) Application number: **18151839.0**

(22) Date of filing: **16.01.2018**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD TN**

(71) Applicant: **Koninklijke Philips N.V.
5656 AE Eindhoven (NL)**

(72) Inventors:
• **KOOIJMAN, Gerben
5656 AE Eindhoven (NL)**
• **RMAILE, Amir Hussein
5656 AE Eindhoven (NL)**
• **GLASSE, Carl
5656 AE Eindhoven (NL)**

• **DE JAGER, Marinus Karel Johannes
5656 AE Eindhoven (NL)**
• **VAN HARTSKAMP, Michael Alex
5656 AE Eindhoven (NL)**
• **CHAPPLE, Iain Leslie Campbell
5656 AE Eindhoven (NL)**
• **GRANT, Melissa Mackay
5656 AE Eindhoven (NL)**
• **PRESHAW, Philip
5656 AE Eindhoven (NL)**
• **TAYLOR, John J.
5656 AE Eindhoven (NL)**

(74) Representative: **de Haan, Poul Erik et al
Philips International B.V.
Philips Intellectual Property & Standards
High Tech Campus 5
5656 AE Eindhoven (NL)**

(54) **PERIODONTITIS DIAGNOSTIC METHODS, USES AND KITS**

(57) Disclosed is an *in vitro* method for assessing whether a human patient has periodontitis. The method is based on the insight to determine biomarker proteins. Accordingly, in a sample of saliva a patient suffering from periodontitis, the concentrations are measured of the Free Light Chain κ protein and/or the Free Light Chain λ. Based on the concentrations as measured, a value is determined reflecting the concentration or joint concentrations for said protein or proteins. This value is compared with a threshold value reflecting in the same manner the concentration or joint concentrations associated with periodontitis. The comparison allows assessing whether the testing value is indicative of the presence of periodontitis in said patient. Thereby, typically, a testing value reflecting a concentration or joint concentration below the concentration or joint concentration reflected by the threshold value is indicative for absence of periodontitis in said patient, and a testing value reflecting a concentration or joint concentration at or above the concentration or joint concentration reflected by the threshold value, is indicative for periodontitis in said patient.

FIG. 1

**Description**

FIELD OF THE INVENTION

The invention is in the field of oral care, and pertains to saliva-based diagnostics of periodontal disease. Particularly, the invention pertains to a kit, use and method for diagnosing periodontitis.

BACKGROUND OF THE INVENTION

Gum inflammation, or gingivitis, is a non-destructive periodontal disease caused mainly by the adherence of dental bacterial biofilms, or dental plaque, to the tooth surface. If not detected and treated, the reversible gingivitis usually leads to the inflammation of the tissues surrounding the tooth (i.e. periodontal tissues), a condition defined as periodontitis, which is irreversible and causes tissue destruction and alveolar bone loss, and ultimately results in the loss of teeth. During the progression of gum disease, there are usually clinical signs and symptoms associated with it, such as the swelling of the gums, the change in color from pink to dark red, the bleeding of the gums, bad breath, and the gums becoming more tender or painful to touch.

Periodontitis is a chronic multifactorial inflammatory disease caused by oral microorganisms and characterized by progressive destruction of the hard (bone) and soft (periodontal ligament) tissues, ultimately leading to tooth mobility and loss. This is to be distinguished from gingivitis which is a reversible infection and inflammation of the gum tissues. Inflammatory periodontitis is one of the most prevalent chronic human diseases and a major cause of adult tooth loss. In addition to the substantial negative impact of periodontitis on oral health, there is also mounting evidence that periodontitis has systemic consequences and that it is a risk factor for several systemic diseases, including heart diseases (e.g. atherosclerosis, stroke), diabetes, pregnancy complications, rheumatoid arthritis and respiratory infections.

Early and accurate diagnosis of periodontal disease, thus, is important from both an oral and overall health perspective.

Periodontal diseases are still poorly diagnosed in general dental practice, resulting in relatively low rates of therapeutic intervention and significant amounts of untreated cases. Current diagnosis relies on imprecise, subjective clinical examination of oral tissue condition (color, swelling, extent of bleeding on probing, probing pocket depth; and bone loss from oral x-rays) by dental professionals. These conventional methods are time consuming, and some of the techniques used (pocket-depth, x-ray) reflect historic events, such as past disease activity, rather than current disease activity or susceptibility to further disease. Hence, more objective, faster, accurate, easier-to-use diagnostics which preferably may also be performed by non-specialists are desirable. Thereby it is desirable to measure current disease activity, and possibly a subject's susceptibility to further periodontal disease.

Saliva or oral fluids have long been advocated as a diagnostic fluid for oral and general diseases, and with the advent of miniaturized biosensors, also referred to as lab-on-a-chip, point of care diagnostics for rapid chair-side testing have gained greater scientific and clinical interest. Especially for periodontal disease detection, inflammatory biomarkers associated with tissue inflammation and breakdown may easily end up in saliva due to proximity, suggesting saliva has strong potential for periodontal disease detection. Indeed, this area thus has gained significant interest and encouraging results have been presented. For example, Ramseier et al (J Periodontol. 2009 Mar;80(3):436-46) identified host- and bacterially derived biomarkers correlated with periodontal disease. However, no definite test has emerged yet.

Biomarkers represent biological indicators that underpin clinical manifestations, and as such are objective measures by which to diagnose clinical outcomes of periodontal disease. Ultimately, proven biomarkers could be utilized to assess risk for future disease, to identify disease at the very earliest stages, to identify response to initial therapy, and to allow implementation of preventive strategies.

Previous limitations to the development of point-of-care tests for salivary biomarkers included a lack of technologies that were adaptable to chair-side applications and an inability to analyze multiple biomarkers in individual samples. Also the selection of which multiple biomarkers to include in such a test has not been adequately addressed in the literature nor implemented in practical tests.

It would be desired to provide a simpler process, and particularly a process that requires only that a small saliva sample is taken from a patient, and possibly by the patient him- or herself. It is desired that such a sample be entered into an *in vitro* diagnostic device, which will allow, based on measurement, a classification of the saliva sample such that it can return an indication of the likelihood that the patient is to be classified as suffering from periodontitis.

SUMMARY OF THE INVENTION

In order to better address the foregoing desires, the invention, in one aspect, concerns an *in vitro* method for assessing whether a human patient has periodontitis, the method comprising detecting, in a sample of saliva from said human patient, the concentrations of the Free Light Chain $\kappa$ protein and/or the Free Light Chain $\lambda$ protein; determining

a testing value reflecting the concentration or joint concentrations determined for said protein or proteins; comparing the testing value with a threshold value reflecting in the same manner the concentration or joint concentrations associated with periodontitis, so as to assess whether the testing value is indicative for periodontitis in said patient.

In another aspect, the invention presents the use of the Free Light Chain κ protein and/or the Free Light Chain λ in a saliva sample of a human patient, as biomarkers for assessing whether the patient has periodontitis.

Optionally, the age of the patient is also used as a biomarker.

In a further aspect, the invention resides in a system for assessing whether a human patient has periodontitis, the system comprising:

detection means able and adapted to detect in a sample of saliva of the human patient the Free Light Chain κ protein and/or the Free Light Chain λ; and

a processor able and adapted to determine from the determined concentrations of said proteins an indication of the patient having periodontitis.

The system optionally contains a data connection to an interface, particularly a graphical user interface, capable of presenting information, preferably also capable of putting in information, said interface being either a part of the system or a remote interface.

Optionally one or more of the foregoing items, particularly the processor, are enabled to function "in the cloud", i.e., not on a fixed machine, but by means of an internet-based application.

In a still further aspect, the invention provides a kit for detecting at least one biomarker for periodontitis in a sample of saliva of a human patient, said kit comprising one or two detection reagents for detecting Free Light Chain κ protein and or Free Light Chain λ. Typically, two detection reagents are used, each of which binds a different biomarker. In one embodiment, a first detection reagent is capable of binding Free Light Chain κ, and a second detection reagent is capable of binding Free Light Chain λ.

In yet another aspect, the invention provides an *in vitro* method for determining a change in status of periodontitis in a human patient over a time interval from a first time point $t_1$ to a second time point $t_2$, the method comprising detecting, in at least one sample of saliva obtained from said patient at $t_1$ and in at least one sample of saliva obtained from said patient at $t_2$, the concentrations of the Free Light Chain λ protein and/or the Free Light Chain κ protein, and comparing the concentrations, whereby a difference in either or both of the concentrations, reflects a change in status.

In a further aspect, the invention provides a method of diagnosing whether a human patient has periodontitis, comprising detecting in a sample of saliva of the human patient the Free Light Chain λ protein and/or the Free Light Chain κ protein, and assessing the presence of periodontitis in the patient on the basis of the concentrations of said protein or proteins in said sample. Optionally, the method of this aspect comprises the further step of treating the periodontitis in the patient.

In yet a further aspect, the invention provides a method of detecting the Free Light Chain λ protein and/or the Free Light Chain κ protein in a human patient, comprising:

(a) obtaining a saliva sample from a human patient; and
(b) detecting whether the Free Light Chain λ protein and/or the Free Light Chain κ protein is present in the sample by contacting the sample with one or more detecting reagents for binding said proteins and detecting binding between each protein and the one or more detecting reagents. Typically, there is a first detection reagent capable of binding the Free Light Chain λ protein, a second detection reagent is capable of binding the Free Light Chain κ protein.

BRIEF DESCRIPTION OF THE DRAWINGS

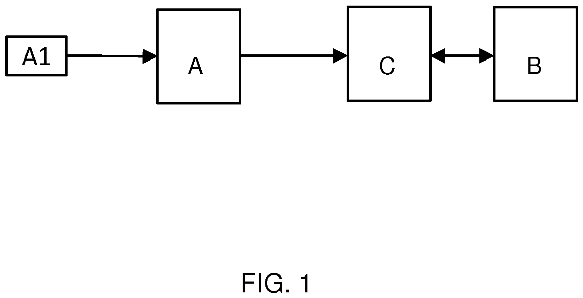

Fig. 1 schematically represents a system for use in the method as described in this disclosure.

DETAILED DESCRIPTION OF THE INVENTION

In a general sense, the invention is based on the judicious insight that even a single protein can serve as a biomarker in a sample of saliva of a human patient, for identifying the presence or absence of periodontitis. Two proteins have been identified that can be used alone or in combination to diagnose periodontitis.

These proteins are the Free Light Chain λ protein and/or the Free Light Chain κ protein. When a single biomarker protein is used, Free Light Chain λ is preferred.

When both proteins are detected, the sum of, ratio of, or difference between, the two proteins can be determined in certain embodiments. The sum, ratio and/or difference can optionally be used in combination with one another. For example, the ratio and sum of the two chains may be combined according to one embodiment, or the ratio and the difference may be combined in another embodiment. The sum, ratio, difference or combination thereof can optionally be further combined with the measures of Free Light Chain λ protein and/or the Free Light Chain κ protein. For example: the κ/λ ratio may be usefully combined with the measure of Free Light Chain λ; the κ/λ ratio may be usefully combined with the measure of Free Light Chain κ; or the κ/λ ratio may be usefully combined with the measure of Free Light Chain κ and Free Light Chain λ.

The subject's age may optionally be included as an additional marker.

Free Light Chain proteins are immunoglobulin light chains. They are not associated with an immunoglobulin heavy chain. Unlike a typical whole immunoglobulin molecule, a Free Light Chain protein is not covalently linked to an immunoglobulin heavy chain, e.g. the Free Light Chain is not disulphide bonded to a heavy chain. Typically the Free Light Chain comprises approximately 220 amino acids. Typically, the Free Light Chain protein comprises a variable region (often referred to as the Light Chain variable Region, $V_L$) and a constant region (often referred to as the Light Chain constant Region, $C_L$). Humans produce two types of immunoglobulin light chains, named with the letter kappa (κ) and lambda (λ). Each of these can be further divided into sub-groups based on variation in the variable region, with four kappa subtypes (Vκ1, Vκ2, Vκ3 and Vκ4) and six lambda subtypes (Vλ1, Vλ2, Vλ3, Vλ4, Vλ5 and Vλ6). Free Light Chain κ is typically monomeric. Free Light Chain λ is typically dimeric, linked by disulphide bonding (to another Free Light Chain λ). Polymeric forms of Free Light Chain λ and of Free Light Chain κ have been identified. Free light chains are produced by bone marrow and lymph node cells as well as locally in the periodontium by diffuse lymphocytes, and are rapidly cleared from the blood and catabolized by the kidneys. Monomeric free light chains are cleared in 2-4 hours, and dimeric free light chains in 3-6 hours.

The two proteins mentioned above are known in the art. The skilled person is aware of their structure, and of methods to detect them in an aqueous sample, such as a saliva sample. Hereinafter the aforementioned protein biomarkers are collectively referred to as "the biomarker panels of the invention." A biomarker panel of the invention, in one embodiment, consists of the two protein biomarkers identified in the invention, i.e., Free Light Chain λ and Free Light Chain κ. In addition to the biomarker panels of the invention, other biomarkers and or data, such as demographic data (e.g., age, sex) can be included in a set of data applied for the determination of the type of periodontitis.

When other biomarkers are optionally included, the total number of biomarkers (i.e. the biomarker panel of the invention plus other biomarkers) is typically 3, 4, 5 or 6.

However, a desirable advantage of the present invention is that the classification of periodontitis in a patient can be determined by measuring preferably not more than two biomarkers, with the biomarker panel of both Free Light Chain λ and Free Light Chain κ being preferred. Particularly, the determination does not need to involve the use of other data, which advantageously provides a simple and straightforward diagnostic test.

The method, as desired, requires only that a small saliva sample, e.g. a dropsize, is taken from the subject. The size of the sample will typically range of from 0.1 μl to 2 ml, such as 1-2 ml, whereby smaller amounts, e.g., 0.1 to 100 μl can be used for *in vitro* device processing, and whereby taking a larger sample, such as up to 20 ml, such as 7.5 to 17 ml, is also possible.

This sample is entered into an *in vitro* diagnostic device, which measures the concentration(s) of the protein or proteins involved, and which returns a diagnostic outcome, classifying the subject on the basis of a likelihood of having periodontitis.

The ease of use of this invention will make it possible to test the majority of dental patients with periodontitis, or with a high risk for developing periodontitis, on a regular basis (e.g. as part of a regular dental check or even at home). This allows, *inter alia*, detecting the presence of periodontitis soon after it has developed, and thus enables more timely taking oral care measures to prevent periodontitis from advancing. Or, e.g., with patients known to be at high risk for periodontitis, and tested for the first time, the method allows to identify whether the periodontitis has developed. Also, the method can be applied after treatment of a patient previously diagnosed with periodontitis, in order to check whether the periodontitis has been successfully treated. Particularly, the method is also suitable for self-diagnosis, whereby the steps of taking the sample and entering it into a device can be conducted by the patient him- or herself.

The patient may typically be known or suspected to have periodontitis when the invention is carried out to confirm whether the periodontitis is present. In certain embodiments therefore, the method is for assessing whether a human patient, known or suspected to have periodontitis, has periodontitis.

A method of the invention typically comprises detecting the aforementioned protein or proteins making up a biomarker panel of the invention, and optional further biomarker proteins, by using one or more detection reagents.

The "saliva" that is tested according to the invention may be undiluted saliva, which may be obtained by spitting or swabbing, or diluted saliva, which may be obtained by rinsing the mouth with a fluid. Diluted saliva may be obtained by the patient rinsing or swilling their mouth for a few seconds with sterile water (for example 5ml or 10ml) or other suitable fluid, and spitting into a container. Diluted saliva may sometimes be referred to as an oral rinse fluid.

By "detecting" is meant measuring, quantifying, scoring, or assaying the concentration of the biomarker proteins. Methods of evaluating biological compounds, including biomarker proteins, are known in the art. It is recognized that methods of detecting a protein biomarker include direct measurements and indirect measurements. One skilled in the art will be able to select an appropriate method of assaying a particular biomarker protein.

The term "concentration" with respect to the protein biomarkers is to be given its usual meaning, namely the abundance of the protein in a volume. Protein concentration is typically measured in mass per volume, most typically mg/ml, μg/ml or ng/ml, but sometimes as low as pg/ml. An alternative measure is Molarity (or Molar concentration), mol/L or "M". The concentration can be determined by detecting the amount of protein in a sample of known, determined or pre-determined volume.

An alternative to determining the concentration is to determine the absolute amount of the protein biomarker in the sample, or determining the mass-fraction of the biomarker in the sample, for example the amount of the biomarker relative to the total of all other proteins in the sample.

A "detection reagent" is an agent or compound that specifically (or selectively) binds to, interacts with or detects the protein biomarker of interest. Such detection reagents may include, but are not limited to, an antibody, polyclonal antibody, or monoclonal antibody that preferentially binds the protein biomarker.

The phrase "specifically (or selectively) binds" or "specifically (or selectively) immunoreactive with," when referring to a detection reagent, refers to a binding reaction that is determinative of the presence of the protein biomarker in a heterogeneous population of proteins and other biologics. Thus, under designated immunoassay conditions, the specified detection reagent (e.g. antibody) binds to a particular protein at least two times the background and does not substantially bind in a significant amount to other proteins present in the sample. Specific binding under such conditions may require an antibody that is selected for its specificity for a particular protein. A variety of immunoassay formats may be used to select antibodies specifically immunoreactive with a particular protein. For example, solid-phase ELISA immunoassays (enzyme linked immunosorbent assay) are routinely used to select antibodies specifically immunoreactive with a protein (see, e.g., Harlow & Lane, Antibodies, A Laboratory Manual (1988), for a description of immunoassay formats and conditions that can be used to determine specific immunoreactivity). Typically a specific or selective reaction will be at least twice the background signal or noise and more typically more than 10 to 100 times the background.

"Antibody" refers to a polypeptide ligand substantially encoded by an immunoglobulin gene or immunoglobulin genes, or fragments thereof, which specifically binds and recognizes an epitope (e.g., an antigen). The recognized immunoglobulin genes include the kappa and lambda light chain constant region genes, the alpha, gamma, delta, epsilon and mu heavy chain constant region genes, and the myriad immunoglobulin variable region genes. Antibodies exist, e.g., as intact immunoglobulins or as a number of well characterized fragments produced by digestion with various peptidases. This includes, e.g., Fab' and F(ab)'2 fragments. The term "antibody," as used herein, also includes antibody fragments either produced by the modification of whole antibodies or those synthesized de novo using recombinant DNA methodologies. It also includes polyclonal antibodies, monoclonal antibodies, chimeric antibodies, humanized antibodies, or single chain antibodies. "Fc" portion of an antibody refers to that portion of an immunoglobulin heavy chain that comprises one or more heavy chain constant region domains, CH1, CH2 and CH3, but does not include the heavy chain variable region. The antibody may be a bispecific antibody, e.g. an antibody that has a first variable region that specifically binds to a first antigen and a second variable region that specifically binds to a second, different, antigen. Use of at least one bispecific antibody can reduce the number of detection reagents needed.

Diagnostic methods differ in their sensitivity and specificity. The "sensitivity" of a diagnostic assay is the percentage of diseased individuals who test positive (percent of "true positives"). Diseased individuals not detected by the assay are "false negatives." Subjects who are not diseased and who test negative in the assay, are termed "true negatives." The "specificity" of a diagnostic assay is 1 minus the false positive rate, where the "false positive" rate is defined as the proportion of those without the disease who test positive.

The biomarker protein(s) of the invention can be detected in a sample by any means. Preferred methods for biomarker detection are antibody-based assays, protein array assays, mass spectrometry (MS) based assays, and (near) infrared spectroscopy based assays. For example, immunoassays, include but are not limited to competitive and non-competitive assay systems using techniques such as Western blots, radioimmunoassays, ELISA, "sandwich" immunoassays, immunoprecipitation assays, precipitin reactions, gel diffusion precipitin reactions, immunodiffusion assays, fluorescent immunoassays and the like. Such assays are routine and well known in the art. Exemplary immunoassays are described briefly below (but are not intended by way of limitation).

Immunoprecipitation protocols generally comprise lysing a population of cells in a lysis buffer such as RIPA buffer (1% NP-40 or Triton X-100, 1 % sodium deoxycholate, 0.1% SDS, 0.15 M NaCl, 0.01 M sodium phosphate at pH 7.2, 1 % Trasylol) supplemented with protein phosphatase and/or protease inhibitors (e.g., EDTA, PMSF, aprotinin, sodium vanadate), adding an antibody of interest to the cell lysate, incubating for a period of time (e.g., 1-4 hours) at 4°C, adding protein A and/or protein G sepharose beads to the cell lysate, incubating for about an hour or more at 4°C, washing the beads in lysis buffer and resuspending the beads in SDS/sample buffer. The ability of the antibody to immunoprecipitate a particular antigen can be assessed by, e.g., western blot analysis. One of skill in the art would be knowledgeable as to the parameters that can be modified to increase the binding of the antibody to an antigen and decrease the background (e.g., pre-clearing the cell lysate with Sepharose beads).

Western blot analysis generally comprises preparing protein samples, electrophoresis of the protein samples in a polyacrylamide gel (e.g., 8%-20% SDS-PAGE depending on the molecular weight of the antigen), transferring the protein sample from the polyacrylamide gel to a membrane such as nitrocellulose, PVDF or nylon, blocking the membrane in blocking solution (e.g., PBS with 3% BSA or non-fat milk), washing the membrane in washing buffer (e.g., PBS-Tween 20), blocking the membrane with primary antibody (the antibody of interest) diluted in blocking buffer, washing the membrane in washing buffer, blocking the membrane with a secondary antibody (which recognizes the primary antibody, e.g., an anti-human antibody) conjugated to an enzymatic substrate (e.g., horseradish peroxidase or alkaline phosphatase) or radioactive molecule (e.g., 32P or 125I) diluted in blocking buffer, washing the membrane in wash buffer, and detecting the presence of the antigen. One of skill in the art would be knowledgeable as to the parameters that can be modified to increase the signal detected and to reduce the background noise.

ELISAs typically comprise preparing antigen (i.e. the biomarker protein of interest or fragment thereof), coating the well of a 96- well microtiter plate with the antigen, adding the antibody of interest conjugated to a detectable compound such as an enzymatic substrate (e.g., horseradish peroxidase or alkaline phosphatase) to the well and incubating for a period of time, and detecting the presence of the antigen. In ELISAs the antibody of interest does not have to be conjugated to a detectable compound; instead, a second antibody (which recognizes the antibody of interest) conjugated to a detectable compound may be added to the well. Further, instead of coating the well with the antigen, the antibody may be coated to the well. In this case, a second antibody conjugated to a detectable compound may be added following the addition of the antigen of interest to the coated well. One of skill in the art would be knowledgeable as to the parameters that can be modified to increase the signal detected as well as other variations of ELISAs known in the art.

When multiple markers are used, a threshold is determined on the basis of the joint concentrations of these biomarkers. When a single biomarker is used, a threshold is determined on the basis of the concentration of that biomarker. The threshold determines whether a patient is classified as having periodontitis or not. The invention reflects the insight that periodontitis can be detected, with sufficient accuracy based on a measurement of the single biomarker or combination of two biomarkers as indicated above.

This insight supports another aspect, the invention, which is the use of the Free Light Chain $\lambda$ protein and/or Free Light Chain $\kappa$ protein, as a biomarker or biomarkers in a saliva sample of a human patient, for assessing whether the patient has periodontitis.

This use can be implemented in a method as substantially described hereinbefore and hereinafter.

When both Free Light Chain $\lambda$ and Free Light Chain $\kappa$ are detected, the method of the invention comprises determining a testing value reflecting the joint concentrations measured for said proteins. A joint concentration value can be any value obtained by input of the concentrations as determined and an arithmetic operation of these values. This can, e.g., be a simple addition of the concentrations. It can also involve multiplying each concentration with a factor reflecting a desired weight of these concentrations, and then adding up the results. It can also involve multiplying the concentrations with each other, or any combination of multiplication, division, subtraction, exponentiation, and addition. It can further involve raising concentrations to some power. In preferred embodiments, the sum of the two biomarkers is used ($\kappa+\lambda$). In other preferred embodiments, the ratio of the two biomarkers is used (e.g. $\kappa/\lambda$ or $\lambda/\kappa$). In other embodiments, the difference between the two biomarkers is used (e.g. $\kappa-\lambda$ or $\lambda-\kappa$).

Optionally, the testing value reflects the concentration of joint concentrations determined for said protein(s) in combination with the age of the subject.

The resulting joint concentration value is compared with a threshold value reflecting in the same manner the joint concentrations associated with the presence of periodontitis. The comparison allows assessing whether the testing value is indicative of the presence of periodontitis in the patients whose saliva is subjected to the test.

The threshold value can, e.g., be the concentration or joint concentration value, obtained in the same manner on the basis of the concentration(s) determined for the same protein(s) in a reference sample associated with the presence of periodontitis, i.e. in a patient diagnosed with periodontitis. Typically, thereby a value reflecting the same or higher joint concentration is indicative of the presence of periodontitis in a tested patient. Analogously, a value reflecting a lower joint concentration in the saliva of a tested periodontitis patient, indicates that periodontitis is absent. However, it will be understood that it is also possible to calculate a threshold value (e.g. by using a negative multiplier) such that a testing value indicating periodontitis would be below the threshold, and a testing value indicating absence of periodontitis, would be above the threshold.

The threshold value can also be determined on the basis of measuring the concentration(s) of the present biomarker protein(s) in a set of samples, including patients with a known diagnosis of periodontitis and "not" periodontitis. Thereby the measured concentration values can be subjected to statistical analysis, possibly including machine learning methods, allowing to discriminate, with the desired sensitivity and specificity, patients classified as periodontitis and patients classified as not suffering from periodontitis. Therefrom, the desired threshold value can be obtained. On the basis of this threshold value, a sample to be tested can be subjected to the same concentration measurement, and the concentration values are then processed, in the same manner in which the threshold value is obtained, so as to determine a joint concentration value that can be compared with the threshold, thus allowing the tested sample to be classified as having periodontitis or not.

In an interesting embodiment, the concentration or joint concentration value is obtained in the form of a score as follows. A numerical value (protein concentration values in e.g. ng/ml) is assigned to each measurement, and these values are used in a linear or nonlinear combination to calculate a score between zero and one. In the event that the threshold value is determined on the basis of a set of subjects as mentioned above, the score between 0 and 1 is typically calculated with the sigmoid function that takes the joint concentration as input (as shown further on).

When the score exceeds a certain threshold, the method indicates that the patient has periodontitis. The threshold may be chosen based on the desired sensitivity and specificity.

It will be understood that in performing a 'periodontitis classification' on a subject, in accordance with the invention, this is on subjects that can be assumed to be at risk from, or suffering from, periodontitis. This can either be known from e.g. a previously performed diagnosis of periodontitis, though perhaps without ability to differentiate the extent of it, or, e.g., assumed from the subject's oral health condition record.

Clinical definitions as acknowledged in the art are based on the following:

*Gingival Index (GI)*

A full mouth gingival index will be recorded based on the Lobene Modified Gingival Index (MGI) rated on a scale of 0 to 4, where:

0 = absence of inflammation,
1 = mild inflammation; slight change in color little change in texture of any portion of but not the entire margin or papillary gingival unit,
2 = mild inflammation; but involving entire margin or papillary unit,
3 = moderate inflammation; glazing, redness, oedema and/or hypertrophy of margin or papillary unit,
- 4 = severe inflammation; marked redness, oedema and/or hypertrophy of marginal or papillary gingival unit, spontaneous bleeding, congestion, or ulceration].

*Probing depths (PD)*

Probing depths will be recorded to the nearest mm using a manual UNC-15 periodontal probe. Probing depth is the distance from the probe tip (assumed to be at the base of the pocket) to the free gingival margin.

*Gingival recession (REC)*

Gingival recession will be recorded to the nearest mm using a manual UNC-15 periodontal probe. Gingival recession is the distance from the free gingival margin to the cemento-enamel junction. Gingival recession will be indicated as a positive number and gingival overgrowth will be indicated as a negative number.

*Clinical attachment loss (CAL)*

Clinical attachment loss will be calculated as the sum of probing depth + recession at each site.

*Bleeding on probing (BOP)*

Following probing, each site will be assessed for bleeding on probing, if bleeding occurs within 30s of probing, a score of 1 will be assigned for the site, otherwise a score of 0 will be assigned.

The resulting subject group (patient group) definition is as follows, whereby the mild-moderate periodontitis and the advanced periodontitis groups are "periodontitis" relevant to the present invention:

Healthy group (H): PD $\leq$ 3 mm in all sites (but would allow up to four 4 mm pockets at distal of last standing molars), no sites with interproximal attachment loss, GI of $\geq$ 2.0 in $\leq$ 10% sites, %BOP scores $\leq$ 10%;
Gingivitis group (G): GI $\geq$ 3.0 in > 30% of sites, no sites with interproximal attachment loss, no sites with PD > 4 mm ,%BOP scores > 10%;
Mild-moderate periodontitis group (MP): interproximal PD of 5-7 mm, (equating to approximately 2-4 mm CAL) at $\geq$ 8 teeth, %BOP scores > 30%;
Advanced periodontitis group (AP): interproximal PD of $\geq$ 7 mm, (equating to approximately $\geq$ 5 mm CAL) at $\geq$ 12 teeth, %BOP scores > 30%.

In an embodiment, the method of the invention makes use of a system as represented schematically in Fig. 1. The system can be a single apparatus having various device components (units) integrated therein. The system can also have its various components, or some of these components, as separate apparatuses. The components shown in Fig. 1 are a measurement device (A), a graphical user interface (B) and a computer processing unit (C).

As mentioned above, the system of the invention comprises a data connection to an interface, whereby the interface itself can be a part of the system or can be a remote interface. The latter refers to the possibility to use a different apparatus, preferably a handheld apparatus such as a smartphone or a tablet computer, for providing the actual interface. The data connection in such cases will preferably involve wireless data transfer such as by Wi-Fi or Bluetooth, or by other techniques or standards.

The measurement device (A) is configured to receive a saliva sample, for example by putting a drop of saliva on a cartridge (A1), which can be inserted into the device (A). The device can be an existing device that is capable to determine, from the same saliva sample, the concentrations of at least one of the Free Light Chain λ and Free Light Chain κ proteins.

The measurement device (A) should be able to receive a saliva sample, for example by putting a drop of saliva on a cartridge (A1), which can be inserted into the device (A). The device may be an existing device that is capable to determine, from the same saliva sample, the concentrations of at least one of the Free Light Chain λ and Free Light Chain κ proteins.

The processing unit (C) receives numerical values for the protein concentrations from part (A). The unit (C) is provided with software (typically embedded software) allowing it to calculate a score (S) between 0 and 1. The software further includes a numerical value for the threshold (T). If the calculated value (S) exceeds (T), unit (C) will output an indication (I) of 'periodontitis' to the GUI (B), otherwise it will output 'no periodontitis'. A further embodiment may use the specific value of (S) to indicate the certainty with which the indication (I) is made. This can be a probability score, whereby 0.5 is a possible threshold value, and e.g. a score S=0.8 would indicate the probability of periodontitis. Interesting options are:

Based on the score S, one can directly indicate a certainty, i.e. S=0.8 means 80% certainty of periodontitis; or
To make the indication through the definition of a range R1-R2, such that when R1<S<R2, the indication (I) will read 'inconclusive'.

A specific calculation of the score can be implemented, e.g., by means of a sigmoid function applying the following formula:

$$S = \frac{1}{1 + \exp(-(c_0 + \sum_{i=1}^{N} c_i B_i))}$$

Wherein $N$ is the number of proteins/biomarkers used. $c_0$, $c_1$, etc. are coefficients (numerical values) and $B_1, B_2$, etc. are the respective protein concentrations.

Determining of the coefficients can be done by a training procedure:

Select N1 subjects with periodontitis (as identified by a dentist via the current criteria) and N2 subjects without periodontitis (having healthy gums or gingivitis).

Take a saliva sample from each subject and determine the protein concentrations of a combination of biomarkers as explained above.

Define the score S to be 1 for periodontitis, and 0 for no periodontitis.

Fit the sigmoid function to the scores and protein concentration values.

Other regression or machine learning methods (linear regression, neural network, support vector machine) may be used where the score S, is high for periodontitis patients and low for the non-periodontitis controls.

With reference to the aforementioned system, the invention also provides, in a further aspect, a system for assessing whether a human patient has periodontitis, the system comprising:

detection means able and adapted to detect in a sample of saliva of the human patient the Free Light Chain κ protein and/or the Free Light Chain λ protein; As explained above, such means are known, and easily accessible to the skilled person; Typically, there is provided a container for receiving an oral sample of a subject therein, the container provided with the detection means;
a processor able and adapted to determine from the determined concentrations of said proteins an indication of the patient having periodontitis.

- Optionally, the system comprises a user interface (or a data connection to remote interface), particularly a graphical user interface (GUI), capable of presenting information; a GUI is a type of user interface that allows users to interact with electronic devices through graphical icons and visual indicators such as secondary notation, instead of text-based user interfaces, typed command labels or text navigation (none of such interface types being excluded in the present invention); GUIs are generally known, and are used typically in handheld mobile devices such as MP3 players, portable media players, gaming devices, smartphones and smaller household, office and industrial controls; as said, the interface optionally can also be chosen so as to be capable of putting in information, such as, e.g., the age of the subject, sex, BMI (Body Mass Index).

The invention also provides, either separately or as part of the aforementioned system, a kit for detecting at least one biomarker for periodontitis in a sample of saliva of a human patient, said kit comprising one or more detection reagents for detecting the Free Light Chain κ protein and/or the Free Light Chain λ protein. Typically, the kit comprises two detection reagents, each directed to a different biomarker, wherein a first detection reagent is capable of binding the Free Light Chain κ protein, a second detection reagent is capable of binding Free Light Chain λ protein. As discussed above with reference to the method of the invention, the kit may comprise more detection reagents, such as for other proteins. In a preferred embodiment the detection reagents made available in the kit consist of the detection reagents for the selection of two proteins making up a 2-biomarker panel of the invention, as mentioned.

Preferably said kits comprise a solid support, such as a chip, a microtiter plate or a bead or resin comprising said detection reagents. In some embodiments, the kits comprise mass spectrometry probes, such as ProteinChip™.

The kits may also provide washing solutions and/or detection reagents specific for either unbound detection reagent or for said biomarkers (sandwich type assay).

In an interesting aspect, the recognition of a biomarker panel of the invention is applied in monitoring the status of periodontitis in a human patient, over time. Accordingly, the invention also provides an *in vitro* method for determining a change in status of periodontitis in a human patient suffering from periodontitis over a time interval from a first time point $t_1$ to a second time point $t_2$, the method comprising detecting, in at least one sample of saliva obtained from said patient at $t_1$ and in at least one sample of saliva obtained from said patient at $t_2$, the concentrations of the Free Light Chain κ protein and/or the Free Light Chain λ protein, and comparing the concentrations, whereby a difference of preferably at least one, and more preferably both concentrations, reflects a change in status. This difference can be reviewed as a difference in concentrations, thus allowing a direct comparison without first generating a number between 0 and 1, or any other classification. It will be understood that the measurements received at both points in time can also be processed in just the same manner as done when determining the periodontitis status as above.

The invention also provides a method of diagnosing whether a human patient has periodontitis, comprising detecting in a sample of saliva of the human patient the Free Light Chain κ protein and/or the Free Light Chain λ protein. The presence of periodontitis in the patient is typically assessed on the basis of the concentrations of said proteins in said sample. Optionally, the method of this aspect comprises the further step of treating the periodontitis in the patient. This optional treatment step can comprise the administration of known therapeutic agents or dental procedures, or a combination of therapeutic agents and dental procedures. Known therapeutic agents include the administration of antimicrobial-containing agents such as a mouthwash, chip, gel or microsphere. A typical antimicrobial agent for use in treating periodontitis is chlorhexidine. Other therapeutic agents include antibiotics, typically orally-administered antibiotics, and enzyme suppressants such as doxycycline. Known non-surgical therapeutic procedures include scaling and root planing (SRP). Known surgical procedures include surgical pocket reduction, flap surgery, gum grafts or bone grafts.

The invention further provides a method of detecting the Free Light Chain κ protein and/or the Free Light Chain λ protein, in a human patient, comprising:

(a) obtaining a saliva sample from a human patient; and
(b) detecting whether Free Light Chain κ protein and/or the Free Light Chain λ proteins are present in the sample by contacting the sample with one or more detecting reagents for binding said proteins and detecting binding between each protein and the one or more detecting reagents.

The invention will be further illustrated with reference to the following nonlimiting example.

Example:

A clinical study was carried out with 153 subjects with varying periodontal health statuses (identified by clinical assessment by a dental professional via current criteria, e.g. American Academy of Periodontology criteria). 39 of the subjects had healthy gums (H), 35 were diagnosed with gingivitis (G), 41 were diagnosed with mild periodontitis (MP) and 38 with advanced periodontitis (AP), ROC (Receiver-Operator-Characteristic) Area-Under-the Curve (AUC) values were obtained following repeated clinical visits at two independent clinical sites.

Performance of various biomarker combinations were evaluated by means of logistic regression with leave-one-out cross validation (LOOCV), resulting in the preferred biomarker combinations as explained herein.

In statistics, a receiver operating characteristic curve, or ROC curve, is a graphical plot that illustrates the performance of a binary classifier system as its discrimination threshold is varied. The curve is created by plotting the true positive rate (TPR) against the false positive rate (FPR) at various threshold settings. The true-positive rate is also known as sensitivity, recall or *probability of detection* in machine learning. The false-positive rate is also known as the fall-out or *probability of false alarm* and can be calculated as (1 - specificity). The ROC curve is thus the sensitivity as a function of fall-out. In general, if the probability distributions for both detection and false alarm are known, the ROC curve can be generated by plotting the cumulative distribution function (area under the probability distribution from - ∞ to the discrimination threshold) of the detection probability on the y-axis, versus the cumulative distribution function of the false-alarm probability on the x-axis. The accuracy of the test depends on how well the test separates the group being tested into those with and without the disease in question. Accuracy is measured by the area under the ROC curve. An area of 1 represents a perfect test; an area of 0.5 represents a worthless test. A guide for classifying the accuracy of a diagnostic test is the traditional academic point system:

0.90-1 = excellent (A)
0.80-0.90 = good (B)
0.70-0.80 = fair (C)
0.60-0.70 = poor (D)
0.50-0.60 = fail (F)

Based on the foregoing, in the results of the aforementioned clinical study, an ROC AUC value of above 0.75 is considered to represent a desirable accuracy for providing a diagnostic test in accordance with the invention.

Table 1 below indicates that Receiver-Operator-Characteristic Area-Under-the Curve values of >0.8 for detecting periodontitis, were obtained using the claimed panels biomarkers.

The biomarker proteins presented in the table are FLC κ, FLC λ, the ratio κ/λ, the sum κ + λ, and the difference κ - λ.

In the table, it can be seen that maximum results, in terms of ROC AUC of 0.847 are obtained with biomarker panel consisting of the ratio κ/λ, the sum κ + λ, and age. A result close to this, at 0.835, is obtained with FLC κ, FLC λ, the ratio κ/λ and age. 0.835 is also obtained with FLC λ, the ratio κ/λ and age. Scores above 0.8 are reported for a number of other markers, including FLC λ alone.

Table 1

| Age | Kappa | Lambda | KL sum | KL ratio | KL diff | AUC LOOCV |
|---|---|---|---|---|---|---|
| Single marker (plus optionally age) | | | | | | |
| | *X* | | | | | *0.688* |
| X | X | | | | | 0.794 |
| | | X | | | | 0.807 |
| X | | X | | | | 0.838 |
| Both κ and λ (plus optionally age) | | | | | | |
| | X | X | X | | | 0.805 |
| X | X | X | | | | 0.830 |
| | X | | | X | | 0.810 |
| X | X | | | X | | 0.850 |
| | | X | | X | | 0.802 |
| X | | X | | X | | 0.835 |
| | | | | X | | *0.558* |
| X | | | | X | | 0.807 |
| | X | X | | X | | 0.805 |
| X | X | X | | X | | 0.835 |

**EP 3 511 716 A1**

(continued)

| Both κ and λ (plus optionally age) | | | | | | |
|---|---|---|---|---|---|---|
| | | | X | | | 0.752 |
| X | | | X | | | 0.821 |
| | | | | | *X* | *0.522* |
| X | | | | | X | 0.769 |
| | | | X | X | | 0.810 |
| X | | | X | X | | 0.847 |
| | | | | X | X | 0.792 |
| X | | | | X | X | 0.825 |

This table shows the performance of FLC biomarker panels, with optionally age added as marker, for classifying periodontitis versus non-periodontitis by means of logistic regression.

Next to the markers κ and λ, additional predictors κ+λ, κ-λ, κ/λ were considered. Redundant panels are not shown in the table. The term redundant here reflects the fact that a panel including e.g. κ+λ and κ-λ as predictors gives -in the logistic regression- the same result as the corresponding panel including κ and λ as predictors.

The panels indicated in italics would not be preferred due to the lower relative performance. All other panels show acceptable performance with AUC LOOCV>0.75 (for most panels AUC LOOCV >0.8).

While the invention has been illustrated and described in detail in the drawings and foregoing description, such illustration and description are to be considered illustrative or exemplary and not restrictive; the invention is not limited to the disclosed embodiments. For example, it is possible to present detection reagents for different biomarkers in different units. Or, conveniently, a kit of the invention can comprise a fixed set of detection reagents for the protein biomarkers that are used in all embodiments, i.e., the Free Light Chain κ protein and/or the Free Light Chain λ protein, and optionally flexible modules comprising a detection reagent for other biomarkers.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain features of the invention are recited in mutually different dependent claims does not indicate that a combination of these features cannot be used to advantage. Any reference signs in the claims should not be construed as limiting the scope.

In sum, we hereby disclose an *in vitro* method for assessing whether a human patient is suffering from periodontitis. The method is based on the insight to determine biomarker proteins. Accordingly, in a sample of saliva from a patient, the concentrations are measured of the Free Light Chain κ protein and/or the Free Light Chain λ protein. Based on the concentrations as measured, a value is determined reflecting the concentration or joint concentrations for said proteins. This value is compared with a threshold value reflecting in the same manner the concentration or joint concentrations associated with periodontitis. The comparison allows assessing whether the testing value is indicative of the presence of periodontitis in said patient. Thereby, typically, a testing value reflecting a concentration or joint concentration below the concentration or joint concentration reflected by the threshold value is indicative for the absence of periodontitis in said patient, and a testing value reflecting a concentration or joint concentration at or above the concentration or joint concentration reflected by the threshold value, is indicative for periodontitis in said patient.

**Claims**

1. An *in vitro* method for assessing whether a human patient has periodontitis, wherein the method comprises:

    detecting, in a sample of saliva from said human patient, the concentrations of the Free Light Chain κ protein and/or the Free Light Chain λ protein;
    determining a testing value reflecting the joint concentrations determined for said protein or proteins;
    comparing said testing value with a threshold value reflecting in the same manner the concentration or joint concentrations associated with periodontitis, so as to assess whether the testing value is indicative for periodontitis in said patient.

2. A method according to claim 1, wherein the human patient is suspected to have periodontitis.

3. A method according to claim 1 or claim 2, wherein the age of the subject is determined and the testing value reflects the concentration or joint concentrations determined for said protein or proteins, in combination with the age of the subject.

4. A method according to any preceding claim, wherein the threshold value is based on the concentration or concentrations determined for the proteins in one or more reference samples each sample associated with the presence of periodontitis or absence of periodontitis.

5. A method according to any of claims 1 to 3, wherein the threshold value is based on the concentration or concentrations of the proteins in a set of samples, including samples from subjects that have periodontitis and samples from subjects not having periodontitis.

6. A method according to any one of the preceding claims, wherein the proteins consist of Free Light Chain κ protein and the Free Light Chain λ.

7. A method according to any one of the claims 1 to 5, wherein the protein is the Free Light Chain λ.

8. A method according to any one of the preceding claims, wherein the concentration values determined are arithmetically processed into a number between 0 and 1.

9. The use of the proteins Free Light Chain κ protein and/or the Free Light Chain λ in a sample of saliva of a human patient, as biomarkers for assessing whether the patient has periodontitis.

10. The use according to claim 9, wherein the age of the human patient is also used as a biomarker.

11. A system for assessing whether a human patient has periodontitis, the system comprising:

   detection means able and adapted to detect in a sample of saliva of the human patient the Free Light Chain κ protein and/or the Free Light Chain λ;
   a processor able and adapted to determine from the determined concentrations of said proteins an indication of the patient having periodontitis.

12. A system according to claim 11, further comprising a container for receiving an oral fluid sample, the container comprising the detection means.

13. A system according to claim 11 or 12, further comprising:

   a user interface for presenting the indication to a user; and
   a data connection between the processor and the user interface for transferring the indication from the processor to the user interface.

14. A system according to any one of claims 11 to 13, wherein the processor is enabled to function by means of an internet-based application.

15. A system according to any of claims 11 to 14, wherein the interface is capable of putting in information on the age of the subject and the processor is able and adapted to determine from the determined concentration or concentrations, an indication that the patient has periodontitis.

16. A kit for detecting at least one biomarker for periodontitis in a sample of saliva of a human patient, said kit comprising one or more detection reagents for detecting Free Light Chain κ protein and/or the Free Light Chain λ.

17. A kit according to claim 16, wherein the one or more detecting reagents comprise at least two detection reagents, a first detection reagent for detecting Free Light Chain κ protein and a second detecting reagent for detecting Free Light Chain λ.

18. A kit according to claim 16 or 17, wherein the one or more detecting reagents are contained on a solid support.

**19.** A kit according to any one of the claims 16 to 18, wherein the one or more detecting reagents consist of detecting reagents for Free Light Chain κ protein and the Free Light Chain λ

**20.** An *in vitro* method for determining a change in status of periodontitis in a human patient suffering from periodontitis over a time interval from a first time point $t_1$ to a second time point $t_2$, the method comprising detecting, in at least one sample of saliva obtained from said patient at $t_1$ and in at least one sample of saliva obtained from said patient at $t_2$, the concentrations of the Free Light Chain κ protein and/or the Free Light Chain λ, and comparing the concentrations, whereby a difference in any one or both of the concentrations, reflects a change in status.

**21.** A method of diagnosing whether a human patient has periodontitis, comprising detecting in a sample of saliva of the human patient the Free Light Chain κ protein and/or the Free Light Chain λ, and assessing the presence of periodontitis in the patient on the basis of the concentration or concentrations of said proteins in said sample.

**22.** A method of detecting the Free Light Chain κ protein and/or the Free Light Chain λ protein, in a human patient, comprising:

(a) obtaining a saliva sample from a human patient; and
(b) detecting whether Free Light Chain κ protein and/or the Free Light Chain λ protein are present in the sample by contacting the sample with one or more detecting reagents for binding said proteins and detecting binding between each protein and the one or more detecting reagents.

A1 → A → C ↔ B

FIG. 1

Europäisches
Patentamt

European
Patent Office

Office européen
des brevets

## EUROPEAN SEARCH REPORT

Application Number

EP 18 15 1839

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CARLO BERTOLDI ET AL: "Non-bacterial protein expression in periodontal pockets by proteome analysis", JOURNAL OF CLINICAL PERIODONTOLOGY, vol. 40, no. 6, 1 June 2013 (2013-06-01), pages 573-582, XP055454955, DK ISSN: 0303-6979, DOI: 10.1111/jcpe.12050 * the whole document * | 1-22 | INV. G01N33/68 |
| X | WO 2014/037924 A2 (KONINKL PHILIPS NV [NL]) 13 March 2014 (2014-03-13) * pp. 2-3, "Brief summary; pp. 11-44, Examples, biomarkers "IPI00972963.1", "IPI00977041.1", "IPI00977405.1"; claims 1-16 * | 1-22 | |
| X | CHIARA BALDINI ET AL: "Proteomic analysis of saliva: a unique tool to distinguish primary Sjägren's syndrome from secondary Sjägren's syndrome and other sicca syndromes", ARTHRITIS RESEARCH AND THERAPY, BIOMED CENTRAL, LONDON, GB, vol. 13, no. 6, 25 November 2011 (2011-11-25), page R194, XP021094475, ISSN: 1478-6354, DOI: 10.1186/AR3523 * the whole document * | 11-15,22 | TECHNICAL FIELDS SEARCHED (IPC) G01N |
| X | US 2006/160233 A1 (JACKOWSKI GEORGE [CA] ET AL) 20 July 2006 (2006-07-20) * p. 5, paragraph [0051] - p. 7, paragraph [0086], pp. 9-10, paragraph [0168] * | 11-15,22 | |

-/--

The present search report has been drawn up for all claims

| Place of search | Date of completion of the search | Examiner |
|---|---|---|
| Munich | 28 February 2018 | R. von Eggelkraut-G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt
European
Patent Office
Office européen
des brevets

**EUROPEAN SEARCH REPORT**

Application Number

EP 18 15 1839

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | RAUBENHEIMER E ET AL: "Salivary immunoglobulin related proteins in 24 patients with multiple myeloma", EUROPEAN JOURNAL OF CANCER. PART B, ORAL ONCOLOGY, PERGAMON, OXFORD, GB, vol. 29, no. 4, 1 January 1993 (1993-01-01), pages 295-297, XP022645576, ISSN: 0964-1955, DOI: 10.1016/0964-1955(93)90051-F [retrieved on 1993-01-01] * the whole document * | 11-15,22 | |
| X | BASILE UMBERTO ET AL: "Free light chains: Eclectic multipurpose biomarker", JOURNAL OF IMMUNOLOGICAL METHODS, vol. 451, 18 September 2017 (2017-09-18), pages 11-19, XP085287882, ISSN: 0022-1759, DOI: 10.1016/J.JIM.2017.09.005 * the whole document * | 11-15,22 | |

TECHNICAL FIELDS
SEARCHED (IPC)

The present search report has been drawn up for all claims

| Place of search | Date of completion of the search | Examiner |
|---|---|---|
| Munich | 28 February 2018 | R. von Eggelkraut-G. |

## ANNEX TO THE EUROPEAN SEARCH REPORT
## ON EUROPEAN PATENT APPLICATION NO.

EP 18 15 1839

This annex lists the patent family members relating to the patent documents cited in the above-mentioned European search report.
The members are as contained in the European Patent Office EDP file on
The European Patent Office is in no way liable for these particulars which are merely given for the purpose of information.

28-02-2018

| Patent document cited in search report | | Publication date | Patent family member(s) | | Publication date |
|---|---|---|---|---|---|
| WO 2014037924 | A2 | 13-03-2014 | CA | 2884089 A1 | 13-03-2014 |
| | | | CN | 104620110 A | 13-05-2015 |
| | | | EP | 2893353 A2 | 15-07-2015 |
| | | | JP | 2015529333 A | 05-10-2015 |
| | | | KR | 20150052311 A | 13-05-2015 |
| | | | RU | 2015113089 A | 27-10-2016 |
| | | | US | 2015219665 A1 | 06-08-2015 |
| | | | WO | 2014037924 A2 | 13-03-2014 |
| US 2006160233 | A1 | 20-07-2006 | AU | 2002336843 A1 | 10-06-2003 |
| | | | JP | 2005510575 A | 21-04-2005 |
| | | | US | 2006160233 A1 | 20-07-2006 |
| | | | WO | 03046572 A2 | 05-06-2003 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **RAMSEIER et al.** *J Periodontol.,* March 2009, vol. 80 (3), 436-46

- **HARLOW ; LANE.** Antibodies, A Laboratory Manual. 1988